# EUROPEAN PATENT SPECIFICATION

(11) **EP 3 530 280 B1**
(45) Date of publication and mention of the grant of the patent: **13.05.2020**
(21) Application number: 17821666.9
(22) Date of filing: 18.10.2017
(51) Int. Cl.: A61P 15/08, A61K 38/09, A61K 31/522

(54) **VETERINARY COMPOSITION FOR MANUFACTURING A SEMINAL ADDITIVE USEFUL FOR ARTIFICIAL INSEMINATION IN ANIMALS**
ZUSAMMENSETZUNG ZUR HERSTELLUNG EINES SAMEN-ADDITIVS ZUR VERWENDUNG BEI KÜNSTLICHER BESAMUNG
COMPOSITION VÉTÉRINAIRE POUR LA FABRICATION D'UN ADDITIF SÉMINAL UTILE POUR L'INSÉMINATION ARTIFICIELLE CHEZ DES ANIMAUX

(30) Priority: 30.11.2016 ES 201631536
(43) Date of publication of application: 28.08.2019
(73) Proprietor: Universidad De León, 24071 León (ES)
(72) Inventor: DOMINGUEZ FERNANDEZ DE TEJERINA, Juan Carlos, 24071 Leon (ES); ALEGRE GUTIERREZ, Beatriz, 24071 Leon (ES); GONZALEZ MONTAÑA, Jose Ramiro, 24071 Leon (ES); ALONSO DE LA VARGA, Marta Elena, 24071 Leon (ES); MARTINEZ PASTOR, Felipe, 24071 Leon (ES)
(74) Representative: Carvajal y Urquijo, Isabel
(86) International application number: PCT/ES2017/070691
(87) International publication number: WO 2018/100214

(56) References cited:
- DATABASE WPI Week 200377 Thomson Scientific, London, GB; AN 2003-827884 XP002777651, BARANOV V. I. ET AL.: "method for treating postpartum diseases in animals, comprising injection of a preparation containing sinestrol, oxytocin, sodium caffeine benzoate and lidocaine", & RU 2 212 233 C2 (N CAUCASUS AREA VETERINARY INST) 20 September 2003 (2003-09-20)
- DATABASE WPI Week 199951 Thomson Scientific, London, GB; AN 1999-591677 XP002777652, LIDICKY J. ET AL.: "polymer conjugates of lecirelin with protracted effect has solution which contains application of lecirelin with protracted effect as veterinary medicine for influencing reproductive cycel of farm animals", & CZ 9 800 950 A3 (BIOPHARM VU BIOFARMACIE A VETERINARNICH) 13 October 1999 (1999-10-13)
- JUSZCZAK M ET AL: "Hypothalamic gonadotropin-releasing hormone receptor activation stimulates oxytocin release from the rat hypothalamo-neurohypophysial system while melatonin inhibits this process", BRAIN RESEARCH BULLETIN, ELSEVIER SCIENCE LTD, OXFORD, GB, vol. 81, no. 1, 15 January 2010 (2010-01-15), pages 185-190, XP026798575, ISSN: 0361-9230 [retrieved on 2009-10-27]

## Description

### FIELD OF THE INVENTION

The present invention belongs to the technical field of veterinary practice, and more particularly to artificial insemination applied to the swine sector. The invention particularly refers to a composition for manufacturing a seminal additive useful for artificial insemination in animals, concretely in female pigs, in order to enhance fertility and prolificacy.

### BACKGROUND OF THE INVENTION

The object of swine artificial insemination is, with a lower operative cost and a higher saving of time and of working organization, to achieve high rates of gestation and a percentage lower than 15% of repetitions, since each oestrous cycle that is lost, or gestation not attained, has a negative effect in the production efficiency being required, as the birth-new gestation period is increased in 21 additional days.

One of the advantages of artificial insemination is that it may require a higher level of handling than in natural covering. For example, with artificial insemination there are more opportunities for human errors to occur than with natural covering. When breeding boar or swine stallion mounts the female, the semen is not exposed to significant environmental changes, and it is generally deposited into the female more than once, during a period comprising the optimum moment for fertilisation.

However, in artificial insemination it is possible that, while semen is collected, diluted, transported and then artificially deposited, many environmental changes occur. Insemination must be correctly done and in the optimum moment. In order to obtain a high percentage of gestation and numerous litters, detection of oestrus must be carefully and faultlessly performed.

Efficiency of the swine artificial insemination depends both on the detection of the ovulation period and on the insemination protocol. The current artificial insemination protocols are based on applying spermatozoids within the 24h-period prior to ovulation, with the further inconvenience that ovulation in the female pig occurs serially and not simultaneously for all the available oocytes in every cycle.

Artificial insemination involves, almost exclusively, the use of refrigerated semen collected between 1 and 7 days prior to use, although several studies indicate that optimum fertility requires insemination within the 48 hours after seminal collection, whether using short or long preservation diluents.

Seminal freezing would allow eliminating time and space restrictions for using of seminal doses. However, insemination using cryopreserved semen features a reproductive performance lower than that obtained with refrigerated semen, both in terms of birth percentage and of litter size. In fact, acceptable percentages of fertility are only found when insemination is performed within a range of 4 hours before each oocyte maturation.

The damage caused by the cryopreservation process is believed to induce an effect in spermatozoids being similar to capacitation or premature ageing, associated to an increase of DNA fragmentation. Therefore, control of sperm capacitation and premature ageing represents a point of interest for improving fertility associated to the use of swine cryopreserved semen.

Semen stimulants, also known as semen enhancers or additives, are those substances which, while not being necessary for semen preservation, improve fertility and/or prolificacy of semen when applied to the seminal doses at the moment immediately before insemination of the female individual, either acting on the intrinsic characteristics of the semen (increasing motility, favouring sperm capacitation, etc.), or triggering in the inseminated female organism reactions being favourable for the fertilising success of the insemination.

The use of semen enhancers have been described in the state of the art. Seminal dilution media may be classified in three categories: extenders (increase seminal volume), preservatives (preserve and protect semen) and enhancers (improve fertilization conditions and embryo viability). Specifically, in relation to the semen dilution media from this last group, reference is expressly made to two particular substances such as hyaluronidase (favouring gametic conjugation) and oxytocin (stimulating tubal-uterine contractions involved in sperm transport).

In the state of the art, an increase in fertility in cows and sows has been described with these semen enhancers. In the 70s, with the development of "in vitro" fertilisation (IVF), substances such as Methylxanthines or calcium ionophores started to be used so as to promote hyperkinesia and sperm capacitation required for fertilization success, especially with oligospermic and hypokinetic semen. An example of methylxanthine is caffeine, which is of practical usefulness for improving fertilization capacity of the refrigerated or frozen swine semen, improving the motility vigour, by acting as sperm cell activator.

Oxytocic substances are highly important in formulating semen enhancers, in particular it has been described a beneficial effect on fertility improvement in sows after addition thereof to the swine semen, since there is a "lack of coital stimuli" at the moment of inseminating sows, which results in a decrease of oxytocic discharges which the mechanical coital act implies, and thus a decrease in the contractility of the genital tract, reducing the capacity thereof for absorbing semen towards the area where the oocytes are to be met (oviduct).

On the other hand, another one of the groups of compounds being important in artificial insemination is hormones. Most frequently used reproductive hormones include the following groups: gonadotropins, progestins, analogues of the gonadotropin releasing factor (GnRH) and prostaglandins. Generally, it may be said that gonadotropins are indicated for stimulating oestrus, progestins for synchronizing thereof, GnRH analogues for ovulation control and prostaglandins for birth or for improvement of uterine involution processes.

Specifically, the synthetic GnRH are 50 times more powerful than natural ones, with an example being Lecirelin, which is joined more persistently to GnRH Hypophyseal receptors and is characterized by a very high power.

However, there is not a composition in the state of the art that meets the capacity of increasing fertility and prolificacy in swine artificial insemination. It was therefore desirable a composition of semen enhancers providing a higher efficiency of the swine artificial insemination process against natural covering, avoiding the existing drawbacks from the state of the art.

### DESCRIPTION OF THE INVENTION

The present invention solves the problems existing in the state of the art by means of a composition for manufacturing a semen additive comprising a combination of semen enhancers which, applied together, allow lower dosage as compared when used individually, ensuring homogeneous and constant results, with high repeatability, while inducing deseasonalization of the "summer" effect on the drop of fertility, typical of swine (summer infertility syndrome).

The present invention is a veterinary composition for manufacturing a semen additive comprising oxytocin, lecirelin and caffeine.

Said veterinary composition for manufacturing a semen additive comprises (per millilitre) an oxytocin concentration between 1 and 3 UI/ml, lecirelin concentration between 0.2 and 0.8 µg/ml and caffeine at a concentration between 1 and 3 mM.

Preferably, in the composition of the invention, the lecirelin being selected is lecirelin acetate.

The veterinary composition of the invention contains an excipient conferring some "self-sterility" characteristics to the composition. In a preferred embodiment, the excipient is 10% chloretone.

The veterinary composition is presented in an aqueous liquid form.

The veterinary composition can be packed in multi-dose packages, being easily applicable in the usual practice of artificial insemination in animals.

Another aspect of the invention refers to the use of the composition as a semen additive being useful for artificial insemination in animals. The three active substances cited have, each one in particular, different properties which, added together, act efficiently as semen additive in the artificial insemination of animals.

An embodiment is the composition of the invention, wherein said veterinary composition is administered directly in the seminal doses at the moment prior to the artificial insemination of animals.

An embodiment is the composition of the invention, wherein said animals are mammals.

A further embodiment is the composition of the invention, wherein said animals are selected from the group consisting of sows, cows, goats and sheep.

The composition of the invention can be used in cases of low semen concentration, certain cases of asthenospermia, and the semen doses stored during an extended period of time under refrigeration (above two days) where a decrease of fertility is always produced.

### BRIEF DESCRIPTION OF THE DRAWINGS

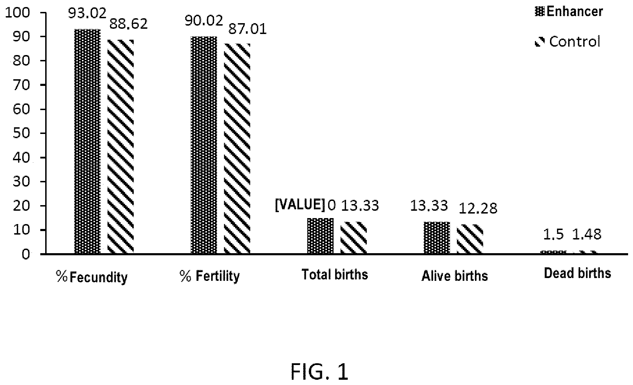

Figure 1 is the effect of the semen enhancer on the fecundity/fertility and prolificacy, in the year 2014, of a group of female individuals receiving the semen enhancer against those not receiving said enhancer (control group).

### PREFERRED EMBODIMENTS

### Example 1. Preparation of the pharmaceutical composition

Batches of 2,000 doses were prepared, wherein each dose is carried in a 1 ml carrier, obtaining total volumes of 2,000 ml. The following products were added into an Erlenmeyer 2,000 ml volumetric flask, perfectly clean and dry:
- 4000 UI of Oxytocin (Facilpart®. Laboratories Syva SAU, 10 U.I/ml presentation).
- 1000 µg of Lecirelin acetate (Dalmarelin®. Fatro Ibérica S.L., 25 µg/ml presentation).
- 38.8 g of Caffein (Caffeine -1,3,7-Trimethylxanthine- Sigma-Chemistry, ref. C0750).
- Excipient (Chloretone at a final concentration of 10%)
and then physiological saline is added until a volume of 2000 ml is reached.

### Example 2. Efficiency of the composition in field studies

In order to examine the effect of the prepared pharmaceutical composition in Example 1 above, for use thereof in swine artificial insemination, field studies were carried out about the effects on fecundity, birth fertility and prolificacy (mean of the total piglets being born (either dead or alive) per delivery). Tables 1 and 2 show the results of the studies being carried out, observing how there are differences being statistically significant in all the experimental groups with respect to control groups.

The statistical characters of fecundity (%fecundity), prolificacy (N.T.), number of alive births (N.V.) and mortality (% of N.M. against N.T.), were tested on factorial ANOVA, LSD Fisher and SAS package (*Statistical Analysis System*).

**Table 1. Effects on fecundity and birth fertility**

| **Exp.** | **Composition** | **Experimental Groups** | | **Control Groups** | |
|---|---|---|---|---|---|
| | | **Fecundity (%)** | **Birth Fertility (%)** | **Fecundity (%)** | **Birth Fertility (%)** |
| **1** | Oxytocin (2 UI) | 90.26 | 88.89 | 83.23 | 82.22 |
| **2** | Synthetic GnRH (0.5 µg) | 91.67 | 89.98 | 92.07 | 87.95 |
| **3** | Pharmaceutical Composition: Oxytocin (2 UI) + Lecirelin acetate (0.5 µg) + Caffeine (2 mM) + Excipient(Chloretone 10%) | 93.02 | 90.02 | 88.62 | 87.01 |

**Table 2. Effects over prolificacy**

| **Exp.** | **Composition** | **Experimental Groups** | **Control Groups** |
|---|---|---|---|
| | | **(Mean ± e.s. (n))** | |
| **1** | Oxytocin (2 UI) | 14.30 ± 0.29 (154) | 12.75 ± 0.30 (161) |
| **2** | Synthetic GnRH (0.5 µg) | 14.80 ± 0.31 (79) | 13.28 ±0.26 (124) |
| **3** | Pharmaceutical Composition: Oxytocin (2 UI) + Lecirelin acetate (0.5 µg) + Caffeine (2 mM) + Excipient (Chloretone 10%) | 14.80 ±0.12 (860) | 13.73 ±0.09 (1824) |

## Claims

1. Veterinary composition, **characterized in that** it comprises oxytocin, lecirelin and caffeine.

2. Veterinary composition according to claim 1, **characterized in that** the oxytocin concentration is between 1 and 3 UI/ml, lecirelin concentration is between 0.2 and 0.8 µg/ml and the caffeine concentration is between 1 and 3 mM.

3. Veterinary composition according to claim 1 or 2, **characterized in that** it comprises lecirelin acetate.

4. Veterinary composition according to claims 1 to 3, **characterized in that** it comprises an excipient.

5. Veterinary composition according to any of claims 1 to 4, **characterized in that** said excipient is 10% chloretone.

6. Veterinary composition according to any of claims 1 to 5, **characterized in that** the composition is in aqueous liquid form.

7. Veterinary composition according to any of claims 1 to 6, **characterized in that** it is packaged in multi-dose containers.

8. Veterinary composition according to any of claims 1 to 7, **characterized in that** it is directly administered in the seminal doses at the moment prior to the insemination.

9. Use of the composition according to any of claims 1 to 8 as a semen additive useful for artificial insemination in animals.

10. Use according to claim 9, **characterized in that** said animals are mammals.

11. Use according to claim 10, **characterized in that** said mammals are selected in the group consisting of sows, cows, goats and sheep.

12. Veterinary composition according to any of claims 1 to 8 for use thereof in artificial insemination in animals with low semen concentration or asthenospermia or for use thereof as an additive at seminal doses stored during an extended period of time under refrigeration.

## Patentansprüche

1. Veterinärmedizinische Zusammensetzung, **dadurch gekennzeichnet, dass** sie Oxytocin, Lecirelin und Koffein enthält.

2. Veterinärmedizinische Zusammensetzung nach Anspruch 1, **dadurch gekennzeichnet, dass** die Oxytocin-Konzentration zwischen 1 und 3 UI/ml, die Lecirelin-Konzentration zwischen 0,2 und 0,8 µg/ml und die Koffein-Konzentration zwischen 1 und 3 mM beträgt.

3. Veterinärmedizinische Zusammensetzung nach Anspruch 1 oder 2, **dadurch gekennzeichnet, dass** sie Lecirelinacetat enthält.

4. Veterinärmedizinische Zusammensetzung nach den Ansprüchen 1 bis 3, **dadurch gekennzeichnet, dass** sie einen Hilfsstoff enthält.

5. Veterinärmedizinische Zusammensetzung nach einem der Ansprüche 1 bis 4, **dadurch gekennzeichnet, dass** der genannte Hilfsstoff Chloreton 10 % ist.

6. Veterinärmedizinische Zusammensetzung nach einem der Ansprüche 1 bis 5, **dadurch gekennzeichnet, dass** die Zusammensetzung in wässriger flüssiger Form vorliegt.

7. Veterinärzusammensetzung nach einem der Ansprüche 1 bis 6, **dadurch gekennzeichnet, dass** sie in Mehrfachdosisbehältern verpackt ist.

8. Veterinärmedizinische Zusammensetzung nach einem der Ansprüche 1 bis 7, **dadurch gekennzeichnet, dass** sie zum Zeitpunkt vor der Besamung in den Samen-Dosierungen direkt verabreicht wird.

9. Verwendung der Zusammensetzung nach einem der Ansprüche 1 bis 8 als Samen-Additiv bei künstlicher Besamung von Tieren.

10. Verwendung nach Anspruch 9, **dadurch gekennzeichnet, dass** die genannten Tiere Säugetiere sind.

11. Verwendung nach Anspruch 10, **dadurch gekennzeichnet, dass** die genannten Säugetiere aus der Gruppe bestehend aus Schweinen, Kühen, Ziegen und Schafen ausgewählt sind.

12. Veterinärmedizinische Zusammensetzung nach einem der Ansprüche 1 bis 8 zur Verwendung bei der künstlichen Besamung von Tieren mit geringer Samenkonzentration oder Asthenospermie oder zur Verwendung als, die während einer längeren Zeitspanne unter Kühlbedingungen gelagert werden.

## Revendications

1. Composition vétérinaire, **caractérisée en ce qu'**elle comprend de l'ocytocine, de la léciréline et de la caféine.

2. Composition vétérinaire selon la revendication 1, **caractérisée en ce que** la concentration en ocytocine est comprise entre 1 et 3 UI/ml, la concentration en léciréline est comprise entre 0,2 et 0,8 µg/ml et la concentration en caféine est comprise entre 1 et 3 mM.

3. Composition vétérinaire selon les revendications 1 ou 2, **caractérisée en ce qu'**elle comprend de l'acétate de léciréline.

4. Composition vétérinaire selon les revendications 1 à 3, **caractérisée en ce qu'**elle comprend un excipient.

5. Composition vétérinaire selon l'une quelconque des revendications 1 à 4, **caractérisée en ce que** ledit excipient est de la chloretone à 10 %.

6. Composition vétérinaire selon l'une quelconque des revendications 1 à 5, **caractérisée en ce que** la composition est sous forme liquide aqueuse.

7. Composition vétérinaire selon l'une quelconque des revendications 1 à 6, **caractérisée en ce qu'**elle est conditionnée dans des récipients multi-doses.

8. Composition vétérinaire selon l'une quelconque des revendications 1 à 7, **caractérisée en ce qu'**elle est administrée directement dans les doses séminales au moment précédant l'insémination.

9. Utilisation de la composition selon l'une quelconque des revendications 1 à 8 comme additif de sperme utile pour l'insémination artificielle chez des animaux.

10. Utilisation selon la revendication 9, **caractérisée en ce que** lesdits animaux sont des mammifères.

11. Utilisation selon la revendication 10, **caractérisée en ce que** lesdits mammifères sont choisis parmi le groupe constitué par les truies, les vaches, les chèvres et les moutons.

12. Composition vétérinaire selon l'une quelconque des revendications 1 à 8 pour son utilisation dans l'insémination artificielle chez des animaux avec une faible concentration de sperme ou de l'asthénospermie ou pour son utilisation comme additif à des doses séminales stockées pendant une période de temps prolongée sous réfrigération.
